# FASCICULE DE BREVET EUROPEEN

(11) **EP 0 384 819 B1**
(45) Date de publication et mention de la délivrance du brevet: **05.10.1994**
(21) Numéro de dépôt: 90400444.7
(22) Date de dépôt: 19.02.1990
(51) Int. Cl.: A61B 17/06

(54) **Suture chirurgicale, notamment pour fermeture de sternotomie**
Chirurgische Naht, insbesondere für Sternotomieverschluss
Surgical suture, especially for sternotomy closure

(30) Priorité: 20.02.1989 FR 8902174
(43) Date de publication de la demande: 29.08.1990
(73) Titulaire: ETHNOR, F-92523 Neuilly sur Seine Cédex (FR)
(72) Inventeur: Prou, Philippe, F-28700 Roinville Sous Auneau (FR)
(74) Mandataire: Martin, Jean-Jacques

(56) Documents cités:
- EP-A- 0 296 776
- GB-A- 2 188 237
- US-A- 2 591 063
- US-A- 4 372 293

## Description

La présente invention concerne les sutures chirurgicales, utilisées dans le cas des interventions nécessitant une sternotomie préalable telles que les interventions à coeur ouvert (fermeture de sternotomie).

Les sutures utilisées pour les interventions de cette nature doivent tout d'abord être conçues pour permettre une pénétration facile des tissus denses malgré leur forte résistance à la pénétration. D'autre part, elles doivent pouvoir assurer une très grande résistance mécanique compte tenu des efforts importants auxquels elles seront soumises pendant une longue durée après l'intervention : tel est typiquement le cas du sternum que l'on a ligaturé pour permettre la reconstitution du tissu osseux entre les deux moitiés disjointes lors de la sternotomie.

Les sutures chirurgicales utilisées jusqu'à présent à cet effet comportent une aiguille courbe très rigide et très acérée à l'une de ses extrémités, prolongée à son autre extrémité par un brin monofil d'acier inoxydable assurant une grande résistance mécanique à la traction. Ce monofil est cependant, en flexion, très déformable plastiquement pour pouvoir suturer convenablement les tissus sur le plan chirurgical. Un exemple d'une telle suture chirurgicale connue comprenant une aiguille fixée sur un monofil d'acier inoxydable est décrit et illustré dans le document GB-A-2188237.

Les sutures de ce type présentent généralement une faiblesse mécanique à l'endroit où le brin monofil est fixé à l'aiguille (généralement par sertissage ou poinçonnage) : en effet, compte tenu de la nature essentiellement rigide de l'aiguille et de la nature essentiellement déformable du monobrin, après plusieurs manipulations de l'aiguille on observe parfois une rupture du monofil à l'endroit où il est serti sur l'aiguille, ou au voisinage de ce point. Cette rupture est causée par la concentration des contraintes au point de liaison entre l'aiguille et le monofil pendant les manipulations.

Pour éviter cet incident, le chirurgien doit prendre garde, lorsqu'il manipule l'aiguille pour réaliser la suture, a ne pas trop solliciter le monofil dans sa partie adjacente à l'aiguille, ce qui peut parfois être difficile compte tenu de la disposition anatomique des organes à suturer et des contraintes de place du champ opératoire.

La présente invention propose une nouvelle suture permettant de remédier à cet inconvénient.

En effet, comme on le verra, la suture de l'invention est, grâce a sa structure, à l'abri de tout risque de rupture en cours de manipulation, tout en étant particulièrement facile à utiliser par le chirurgien ; celui-ci pourra donc exécuter son travail rapidement et sans risque.

A cet effet, la suture de l'invention, qui comprend une aiguille essentiellement rigide, reliée à un monofil, essentiellement déformable plastiquement en flexion, est caractérisée en ce que l'aiguille et le monofil sont reunis par un élément intermédiaire filiforme essentiellement déformable élastiquement en flexion, formé d'un câble multibrin torsadé.

De préférence, l'élément intermédiaire est assemblé à l'aiguille et/ou au monofil par soudure autogène.

Le document US-A-4372293 décrit un système pour chirurgie plastique comprenant une aiguille fixée à une première extrémité d'un ruban dont la seconde extrémité est elle-même fixée à un fil d'acier inoxydable. Ce document ne se préoccupe aucunement des sutures chirurgicales pour fermeture de sternotomie et donc des problèmes spécifiques très particuliers posés par ces sutures compte-tenu de la très grande résistance mécanique requise pour les éléments qui les composent. Par ailleurs, le système proposé dans le document US-A-4372293 constitue plutôt un implant pour chirurgie plastique, qu'une suture chirurgicale.

Le document US-A-2591063 décrit une suture chirurgicale pour chirurgie oculaire.

On va maintenant donner un exemple de réalisation de l'invention.

La figure 1 est une vue de la suture de l'invention montrant les différents éléments constitutifs de celle-ci.

La figure 2 est un détail, repéré II sur la figure 1, de la liaison aiguille-cable multibrin.

La figure 3 est un détail, repéré III sur la figure 1, de la liaison cable multibrin-monofil.

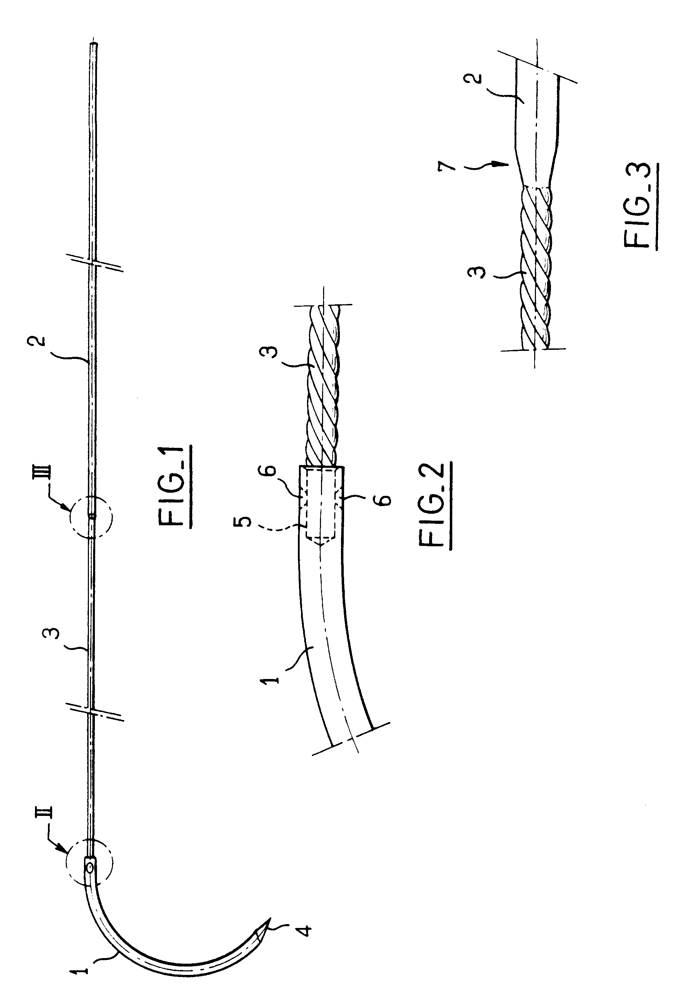

La suture de l'invention (figure 1) comporte une aiguille 1 et un monofil 2 qui sont des éléments en eux-mêmes connus.

L'aiguille 1 est une aiguille présentant une courbure circulaire, réalisée à partir d'un corps rond présentant une extrémité triangulaire 4 très acérée et de section inférieure au corps de l'aiguille, pour diminuer le traumatisme tissulaire.

Cette aiguille est réalisée en un matériau rigide (pour éviter qu'elle ne se déforme) et résistant (pour éviter que la pointe ne s'émousse), par exemple un acier inoxydable ayant subi un traitement thermique approprié et un polissage soigneux.

Le monofil 2 est réalisé en un matériau qui assure une grande résistance mécanique à la traction (faible ductilité), qui permette cependant une déformation plastique aisée afin d'être aisément courbé pour réaliser la suture, et qui soit neutre du point de vue des réactions tissulaires éventuelles.

Il peut être par exemple réalisé à partir d'un fil de diamètre parfaitement calibré d'acier inoxydable d'aspect lisse, parfaitement poli et brillant.

De façon caractéristique de l'invention, au lieu que le monofil et l'aiguille soient directement fixés l'un à l'autre (par sertissage ou poinçonnage) comme c'était le cas dans l'art antérieur, ces deux éléments sont réunis par l'intermédiaire d'un élément intermédiaire filiforme 3 permettant d'assurer la transition, du point de vue des propriétés mécaniques, entre l'aiguille 1 et le monofil 2.

Cet élément intermédiaire 3 est un fil réalisé en un matériau qui est essentiellement déformable élastiquement, ce qui permet d'assurer la transition entre l'aiguille 1, qui est essentiellement rigide, et le monofil 2, qui est essentiellement déformable plastiquement.

Très avantageusement, cet élément intermédiaire 3 est formé d'un câble multibrin torsadé, qui présente plusieurs avantages :
- du fait de sa régularité, son profil extérieur le rapproche le plus possible d'un monofil ;
- il a une résistance à la traction comparable à celle d'un monofil, mais en revanche une souplesse (possibilité de déformation élastique) qui permet toute latitude de mouvement à la manipulation de l'aiguille par le chirurgien ;
- enfin, sa qualité le rend neutre dans l'utilisation à travers les tissus.

Le matériau de ce câble multibrin est avantageusement un acier inoxydable.

La figure 2 montre le détail de l'assemblage entre l'aiguille 1 et le câble multibrin 3.

Pour permettre cet assemblage, l'aiguille 1 est par exemple forée à son extrémité d'un alésage axial borgne 5 de diamètre légèrement supérieur à celui du câble multibrin. Le câble multibrin est alors introduit à l'intérieur de l'alésage 5 puis l'ensemble est présenté sur un poste de soudage autogène, par exemple de soudage par laser YAG, qui réalise deux points de soudure 6 diamétralement opposés dans la zone d'assemblage.

La figure 3 montre le détail de l'assemblage entre le câble multibrin 3 et le monofil 2.

Pour assembler ces deux éléments, on peut par exemple les présenter bout à bout, l'extrémité du monofil étant mise en contact avec le câble multibrin avant d'être immobilisée. On procède alors à la jonction des deux éléments par soudure autogène (ici encore, de préférence par soudure laser), de manière à faire couler la surépaisseur du monofil sur le câble multibrins, cette opération étant exécutée sur tout le pourtour du câble et du monofil.

Comme on peut le voir en 7 sur la figure 3, ce procédé d'assemblage ne provoque ni surépaisseur ni bavure et permet d'obtenir un profil présentant une transition parfaitement régulière et continue entre le profil cylindrique du câble multibrins et celui du monofil.

A l'usage, cette suture s'est révélée particulièrement efficace, notamment en ce qui concerne :
- sa résistance à la traction, qui est notablement supérieure à celle d'une suture traditionnelle avec assemblage par sertissage ou poinçonnage ;
- la diminution des risques de rupture entre l'aiguille et le fil, ce qui permet une traction omnidirectionnelle de l'aiguille sur le fil en toute sécurité.

Les diamètres et des longueurs des différents éléments peuvent être très variables en fonction des applications envisagées. Pour une aiguille de 48 mm de long et de 1,4 mm de diamètre, on peut par exemple prévoir un câble multibrin de 150 mm de long et de 0,74 mm de diamètre, pour un monofil de 450 mm de long et de 0,78 ou 0,88 mm de diamètre.

Ces dimensions ne sont bien sûr données qu'à titre d'exemple, le seul point important étant que le diamètre du câble multibrin soit légèrement inférieur (de préférence, de 0,04 mm au moins) à celui du monofil 2 afin de permettre un soudage convenable, sans surépaisseur.

## Revendications

1. Une suture chirurgicale pour fermeture de sternotomie, comprenant une aiguille (1), essentiellement rigide, reliée a un monofil (2), essentiellement déformable plastiquement en flexion, caractérisée en ce que l'aiguille (1) et le monofil (2) sont réunis par un élément intermédiaire filiforme (3) essentiellement déformable élastiquement en flexion, formé d'un câble multibrin torsadé.

2. La suture de la revendication 1, dans laquelle l'élément intermédiaire (3) est assemblé à l'aiguille (1) par soudure autogène.

3. La suture de l'une des revendications 1 ou 2, dans laquelle l'élément intermédiaire (3) est assemblé au monofil (2) par soudure autogène.

4. La suture de l'une des revendications 1 à 3, caractérisée par le fait que le câble multibrin torsadé (3) est en acier.

5. La suture de l'une des revendications 1 à 4, caractérisée par le fait que le monofil (2) est en acier.

6. La suture de l'une des revendications 1 à 5, caractérisée par le fait que le diamètre du câble multibrin torsadé (3) est inférieur à celui du monofil (2).

7. La suture selon la revendication 6, caractérisée par le fait que le diamètre du câble multibrin (3) est inférieur d'au moins 0,04mm à celui du monofil (2).

8. La suture de l'une des revendications 1 à 7, caractérisée par le fait que l'assemblage du câble multibrin torsadé (3) sur le monofil (2), est réalisé en plaçant ces deux éléments bout à bout, jusqu'à mise en contact et en procédant par soudure laser sur tout le pourtour du câble (3) et du monofil (2), de manière à faire couler la surépaisseur du monofil (2) sur le câble multibrin.

9. La suture de l'une des revendications 1 à 8, caractérisée par le fait que le câble multibrin (3) a une longueur de l'ordre de 150mm tandis que le monofil (2) a une longueur de l'ordre de 450mm.

10. La suture de l'une des revendications 1 à 9, caractérisée par le fait que le diamètre du câble multibrin (3) est de l'ordre de 0,74mm tandis que le diamètre du monofil (2) est de l'ordre de 0,78 à 0,88mm.

11. La suture de l'une des revendications 1 à 10, caractérisée par le fait que le câble multibrin (3) est fixé sur l'aiguille (1) par soudure laser.

12. La suture de l'une des revendications 1 à 11, caractérisée par le fait que l'aiguille (1) est munie d'un alésage axial borgne (5) dans lequel est engagée l'extrémité du câble multibrin (3) et que ce dernier est fixé sur l'aiguille par soudure laser, de préférence sous forme de deux points de soudure (6) diamétralement opposés.

## Claims

1. A surgical suture for sternotomy closure, comprising an essentially rigid needle (1) connected to a monofilament (2) which is essentially plastically deformable in flexion, characterized in that the needle (1) and the monofilament (2) are joined by means of a filiform intermediate element (3) which is essentially elastically deformable in flexion and consists of a twisted multi-strand cord.

2. The suture of Claim 1, in which the intermediate element (3) is attached to the needle (1) by autogenous welding.

3. The suture of one of Claims 1 or 2, in which the intermediate element (3) is attached to the monofilament (2) by autogenous welding.

4. The suture of one of Claims 1 to 3, characterized in that the twisted multi-strand cord (3) is made of steel.

5. The suture of one of Claims 1 to 4, characterized in that the monofilament (2) is made of steel.

6. The suture of one of Claims 1 to 5, characterized in that the diameter of the twisted multi-strand cord (3) is smaller than that of the monofilament (2).

7. The suture of Claim 6, characterized in that the diameter of the multi-strand cord (3) is smaller than that of the monofilament (2), by at least 0.04 mm.

8. The suture of one of Claims 1 to 7, characterized in that the assembly of the twisted multi-strand cord (3) on the monofilament (2) is effected by placing these two elements end to end, until they come into contact, and by carrying out laser welding on the whole periphery of the cord (3) and the monofilament (2), in such a way as to slide the increased thickness of the monofilament (2) over the multi-strand cord.

9. The suture of one of Claims 1 to 8, characterized in that the multi-strand cord (3) has a length of the order of 150 mm, while the monofilament (2) has a length of the order of 450 mm.

10. The suture of one of Claims 1 to 9, characterized in that the diameter of the multi-strand cord (3) is of the order of 0.74 mm, while the diameter of the monofilament (2) is of the order of 0.78 to 0.88 mm.

11. The suture of one of Claims 1 to 10, characterized in that the multi-strand cord (3) is fixed on the needle (1) by laser welding.

12. The suture of one of Claims 1 to 11, characterized in that the needle (1) is provided with a blind axial bore (5) in which the end of the multi-strand cord (3) is engaged, and in that the latter is fixed on the needle by laser welding, preferably in the form of two diametrically opposed welding points (6).

## Patentansprüche

1. Chirurgische Naht für den Sternotomieverschluß mit einer im wesentlichen starren Nadel (1), die mit einem im wesentlichen biegeverformbaren Monofilament (2) verbunden ist, dadurch gekennzeichnet, daß die Nadel (1) und das Monofilament (2) über ein im wesentlichen biegeelastisch verformbares, drahtförmiges Zwischenelement (3) aus einem vielsträngigen, verdrillten Kabel verbunden sind.

2. Naht nach Anspruch 1, bei der das Zwischenelement (3) mittels Autogenschweißen an der Nadel (1) befestigt ist.

3. Naht nach Anspruch 1 oder 2, bei der das Zwischenelement (3) mittels Autogenschweißen am Monofilament (2) befestigt ist.

4. Naht nach einem der Ansprüche 1 bis 3, dadurch gekennzeichnet, daß das vielsträngige, verdrillte Kabel (3) aus Stahl ist.

5. Naht nach einem der Ansprüche 1 bis 4, dadurch gekennzeichnet, daß das Monofilament (2) aus Stahl ist.

6. Naht nach einem der Ansprüche 1 bis 5, dadurch gekennzeichnet, daß das vielsträngige, verdrillte Kabel (3) einen kleineren Durchmesser hat als das Monofilament (2).

7. Naht nach Anspruch 6, dadurch gekennzeichnet, daß der Durchmesser des vielsträngigen Kabels (3) mindestens 0,04 mm kleiner ist als der des Monofilaments (2).

8. Naht nach einem der Ansprüche 1 bis 7, dadurch gekennzeichnet, daß das vielsträngige, verdrillte Kabel (3) an dem Monofilament (2) befestigt wird, indem diese beiden Elemente mit ihren Enden aneinandergelegt werden, bis sie sich berühren, und das Kabel (3) und das Monofilament (2) um ihren gesamten Umfang herum mit einem Laserstrahl so verschweißt werden, daß ein gleichmäßiger Dickenübergang von dem Monofilament (2) auf das vielsträngige Kabel erfolgt.

9. Naht nach einem der Ansprüche 1 bis 8, dadurch gekennzeichnet, daß die Länge des vielsträngigen Kabels (3) im Bereich von 150 mm liegt, während die Länge des Monofilamentes (2) im Bereich von 450 mm liegt.

10. Naht nach einem der Ansprüche 1 bis 9, dadurch gekennzeichnet, daß der Durchmesser des vielsträngigen Kabels (3) im Bereich von 0,74 mm liegt, während der Durchmesser des Monofilamentes (2) im Bereich von 0,88 mm liegt.

11. Naht nach einem der Ansprüche 1 bis 10, dadurch gekennzeichnet, daß das vielsträngige Kabel (3) mittels Laserstrahlschweißen an der Nadel (1) befestigt ist.

12. Naht nach einem der Ansprüche 1 bis 11, dadurch gekennzeichnet, daß die Nadel (1) ein axiales Sackloch (5) aufweist, in dem das Ende des vielsträngigen Kabels (3) in Eingriff genommen ist, und daß letzteres mittels Laserstrahlschweißen an der Nadel befestigt ist, vorzugsweise in Form von zwei sich diametral gegenüberliegenden Schweißstellen (6).
